# EUROPEAN PATENT SPECIFICATION

(11) **EP 0 747 058 B1**
(45) Date of publication and mention of the grant of the patent: **22.09.2004**
(21) Application number: 96112324.7
(22) Date of filing: 26.04.1990
(51) Int. Cl.: A61K 39/10

(54) **Acellular vaccine**
Azellulärer Impfstoff
Vaccine acellulaire

(30) Priority: 08.05.1989 GB 8910570
(43) Date of publication of application: 11.12.1996
(62) Divisional of application: 90907222.5
(73) Proprietor: MEDEVA B.V., 1078 ED Amsterdam (NL)
(72) Inventor: Novotny, Pavel, Bromley, Kent, BR2 0BT (GB)
(74) Representative: Woods, Geoffrey Corlett

(56) References cited:
- EP-A- 0 162 639
- EP-A- 0 267 998
- THE JOURNAL OF EXPERIMENTAL MEDICINE, vol. 168, no. 4, October 1988, NEW YORK, NY, USA, pages 1351-1362, XP002015155 M. DE MAGISTRIS ET AL.: "Dissecting human T cell responses against Bordetella species."

## Description

The present invention relates to acellular Bordetella pertussis vaccine compositions.

Bordetella pertussis causes a serious and debilitating disease in humans, children being particularly susceptible, which is kept under control in the developed countries by large scale immunisation programmes. It has been found that immunisation is a very important factor in the reduction of the disease and that failure to vaccinate can lead to increased incidence of the disease. In practically all areas, immunisation is effected using a whole cell B.pertussis vaccine which has been found to be relatively effective in preventing the disease. However, recently, concern over adverse reactions to the vaccines has led to lower vaccine acceptance and debate about its continued use.
Some of the adverse reactions noted include fever, local reactions and persisiant screaming. The incidence of fever and persistant screaming have been estimated to occur in 7% of patients (Wardlaw et al Medical Microbiology Vol.2. Immunisation against Bacterial Disease 1983).

With the currently low occurrence of the disease in developed countries with immunisation programmes, the benefit/risk ratio is poorly defined, and many clinicians believe that the risk of inoculation outweigh the benefits gained by immunisation. As a result, many children are not inoculated and there is now a consequent risk of a pandemic of whooping cough. Indeed in recent years the incidence of whooping cough and resulting infant morbidity has increased as the use of the whole cell vaccine has decreased. Considerable research effort has, therefore, been directed towards the development of improved pertussis vaccines and especially acellular vaccines which lack the components associated with the toxic effects of the whole cell vaccines which have caused the concerns, whilst incorporating those components necessary to protect against the disease.

The search for, a safer, effective, acellular B.pertussis vaccine has been hampered in the past by the paucity of information regarding the identity and mechanisms of action of the pathogenic, toxic and protective moieties of B.pertussis contained in the whole cell vaccines. Work has, therefore, been concentrated on isolating and purifying the 20 or more surface antigens of the B.pertussis organism and characterising their ability to induce immune reactions (see, for example, J.Am.Med.Soc. 248 (1) 22-23). Examples of antigens that have been suggested for investigation include lymphocytosis promoting factor (pertussis toxin/LPF) filamentous haemagglutinin (FHA), lipopolysaccharide (LPS), agglutinogens, dermonecrotic toxin (DNT), heat labile and heat stable toxins, polymorphonuclear leukocyte inhibitor factor, adenylate cyclase and other surface components. Other proposed candidate antigens for investigation include tracheal cytotoxin and various outer membrane proteins.

An early extract vaccine was developed by L.Pillemer (Proc.Soc.Exp.Biol.Med. (1950) 75, 704-705) which was based on disrupted B.pertussis cells and found to provide protection, but which was not adopted commercially in view of the toxicity of the preparation.

Examples of more recent B.pertussis extract vaccines that have been suggested include those described in UK Patent Specification 2 083 358A (Takeda) involving removal of endotoxin from culture supernatants; French Patent Specification 2 047 886 (Institut Merieux) involving extraction of a microbial suspension with an anionic surfactant; and Japanese Patent Specification 58-222032 (Teijin) which comprises a sub-unit protein vaccine based on pertussis toxin (LPF).

Much of the work carried out on acellular pertussis vaccines is concentrated on the possibility of basing such a vaccine on LPF. However, it is believed that some of the adverse effects hitherto observed to be associated with pertussis vaccination are related to the toxin. In combination-with tetanus or diphtheria toxoid and LPS, it is able to induce experimental encephalopathy in susceptible mice (L.Steinman, et al. Nature (1982) 299, 738-740; Redhead et al, Workshop on B.pertussis, Nat.Inst. of Biol. Standards & Controls, Holy Hill, Hampstead, London, 1983). Thus some clinicians believe that LPF may, possibly, be responsible for brain damage should such complications occur after vaccination.

Nonetheless studies to date, have generated data which has led to a general belief that LPF is an essential part of any acellular vaccine (Bacterial Vaccines, 1984, Chapter 3, Manclark et al, Editor Germanier).

A new acellular vaccine, currently available in Japan has been tested in controlled clinical trials in Sweden. This vaccine includes the pertussis toxin (LPF) and FHA or LPF alone (Lancet 1 995 1988). However this vaccine has proved not to be as effective as a whole cell vaccine, providing only about 69% protection.

Apart from the poor protective effect three deaths occurred in the toxin based vaccine group which may possibly be associated with the vaccine. Considering all these data, the Swedish Health Authority refused to licence this so called "Japanese Vaccine" in Sweden.

This clinical trial, however, is an illustration of the belief that LPF antigen is an essential component of the vaccine since it has been suggested that whooping cough is a toxin-mediated disease and that the protection of mice in the pertussis mouse protection test is solely dependent on the presence of an active LPF in the preparation (Pittman,M. 1984 : The Concept of Pertussis as a Toxin-Mediated Disease, Pediatric Infection Disease, 3. 467-486). It is believed that these assumptions are incorrect.

Filamentous haemagglutinin (FHA) is a protein having a molecular weight of between 107-130 kDa and appears as filaments in the electron microscope. It is a haemagglutin that is inhibited by cholesterol.

Many research groups have suggested that FHA may be an important immunogen and vaccine candidate. (For a review see Bacterial Vaccines 1984. Chapter 3, Manclark et al, Editor Germanier). Our data shows however, that FHA alone only provides minimal protection.

The 69kDa antigen of pertussis is an outer membrane protein that is heat-stable and can be prepared by methods known in the art (see EP0162639). The use of 69kd on its own is not as efficient as the whole cell vaccine.

De Magishis et al. (J. Exp. Med. **168** 1351-1362 (1998)) reports the result of a study to investigate humoral and cellular responses in adults who had suffered from pertussis in childhood T cell clones were obtained.

The present inventor has found that a 69kDa/FHA vaccine is more potent than 69kDa/LPF and at least as, if not more, potent than the whole cell vaccine. The combination of 69kDa and FHA is advantageous since LPF is not required, and consequently the chances of adverse effects are reduced. Additionally, a bivalent vaccine containing only 69kDa and FHA will clearly be easier and cheaper to manufacture than a trivalent vaccine containing LPF as well.

Apart from that, by a proper combination of pertussis antigens the equal effective dose of the suggested combination is up to 15 times lower than, for example, a combination of the 69kDa protein and LPF.

Thus according to the present invention there is provided an acellular vaccine comprising the 69kDa antigen of Bordetella pertussis, the filamentous haemagglutinin antigen of Bordetella pertussis, an adjuvant and a saline solution, the 69kDa antigen and the filamentous haemagglutinin antigen being present in a weight ratio of between 1:10 and 1:1 and the vaccine being at least as potent as British reference whole cell vaccine 66/84 in the Kendrick test.

The ratio of 69kDa antigen to FHA is preferably approximately 1:1.

The vaccine formulation comprises an adjuvant for stimulating the immune response and thereby enhancing the effect of the vaccine. Convenient adjuvants for use in the present invention include, for example, aluminium hydroxide and aluminium phosphate.

Conveniently the vaccine formulations are presented to contain a final concentration of antigenic protein in the range of from 0.01 to 5 mg/ml, preferably 0.03 to 2 mg/ml, most preferably 0.3 mg/ml. After formulation the vaccine may be incorporated into a sterile container which is then sealed and stored at a low temperature, for example 4°C, or may be freeze-dried.

In order to induce immunity in man to whooping cough one or more doses of the vaccine suitably formulated may be administered. It is recommended that each dose is 0.1 to 2 ml, preferably 0.2 to 1 ml, most preferably 0.5 ml of vaccine.

The vaccine may be used in a method of inducing immunity to whooping cough in man. The vaccines according to the present invention may be administered by any conventional method for the administration of vaccines including oral and parenteral. The treatment may consist of a single dose or a plurality of doses over a period of time. The invention includes the use of the 69kDa antigen of Bordetella pertussis, the filamentous haemagglutinin antigen of Bordetella pertussis, an adjuvant and a saline solution for the manufacture of an acellular vaccine for the prophylactic treatment of a mammal susceptible to B.pertussis infection, wherein the vaccine contains the 69kDa antigen and the filamentous haemagglutinin antigen in a weight ratio of between 1:10 and 1:1 and is at least as potent as British reference whole cell vaccine 66/84 in the Kendrick test.

### Example 1

### Preparation of Filamentous Haemagglutinin (FHA)

FHA can be prepared in methods well known in the art (see Araih and Munoz J.J. (1970), Infect.Immunology 25 764-767; Ashworth et al (1982) Infect.Immun 37 1278-1281; Cowell et al Bacterial Vaccines, p371-379 Seminars in Infectious diseases Vol. IV (1982); Sato et al (1983) Infect.Immun. 41 313-320). However FHA in the following procedure was prepared in accordance with the following protocol.

FHA purification: B.pertussis Tomaha or BP357 (Tn5 transposon mutant of AA.Weiss et al (1983) which does not secrete the LPF) or W28ΔLPF obtained from R.Rappuloi were grown in Stainer & Scholte medium (0.05 Tris) in 650ml Costar flasks (150ml, in each) for 5 days at 37C (Sato et al 1983 supra). Before centrifugation (30mins at 6000 x g) 50uM 1,10-phenanthroline monohydrate as proteolysis inhibitor was added to the cultures. The cell free supernatant adjusted to pH 8.7 (using 5N.NaOH) was applied to a 30 x 350mm column of Spheroidal Hydroxylapatite (BHD) at a flow rate of 500ml/hr. (All operations at room temperature). The column was then washed in the cold room (+4^{o}C) until stable baseline was achieved at a flow rate of 50ml/hr with (a) 10mM phosphate buffer, pH 8.0, (b) 100mM phosphate buffer, pH 8.0, and finally (c), the retained material was eluted with 0.5 N.NaCl in 100mM phosphate buffer, pH 7.0. The peak fractions agglutinating goose red blood cells (10µl volumes from each fraction suspended in 50ul of PBS and an equal amount of washed 0.5% goose blood cells were added and incubated for 1-2 hours at 37°C) were pooled. The Pool was dialyzed overnight against 20-30 volumes of 0.025M Bis-Tris/HCl buffer, pH 7.1, at 4°C. The precipitated FHA was collected on a centrifuge (20mins at 8000xg). The next step was inspired by Cowell et al (1983) who found that the FHA (as well as LPF) is soluble in 40mM beta-alanine buffer, pH 3.5. The precipitated FHA was solubilized in the smallest possible volume of β-alanine buffer (3.57g β-alanine and 0.35g formic acid per litre), insolubles removed by centrifugation and the clear supernatant was applied to a column (25 x 800mm) of Ultrogel AcA 34 or Aca 44 equilibrated and eluted with the same buffer to remove impurities. The retained haemagglutinating material appeared in a peak eluted by 0.05M.Tris/HCl buffer containing 0.5M NaCl (pH7.2). The fractions from the main peak and having haemagglutinatious properties were pooled and kept frozen or re-precipitated by dialysis against 0.025M Bis-Tris/HCl buffer, pH 7.1 and dissolved in a smaller volume of β-alanine buffer. The solubility is approx 3.0mg FHA/ml. The final product thus obtained, either from the Tomaha, BP357 or W28 LPF strain does not contain detectable amount of LPF as measured by CHO cell assay (which was negative at a concentration of 2-3ug FHA per single well containing 200ul tissue culture; sensitivity of the test: 1-2pg LPF per well gives a positive clumping) or by histamine sensitization. The N:NIH-S mice were injected with doses of 50ug of FHA intreperitoneally and challenged 4 days later by intraperitoneal injection if 4mg histamine hydrochloride. None of them died. The material frozen. (at -20 or -40°C) at acid pH appears stable as judged from its ELISA reactivity and appearance in the SDS-PAGE: It forms prevalently three strong bands in the region of 150-100 kD.

### Example 2

69kDa antigen was prepared in accordance with the procedures outlined in published European patent application No. 0162 639, using either strain BP357 or U28ΔLPF of B.pertussis, and immunopurified using the monoclonal antibody BB05 - deposited at the Public Health Laboratory Service Centre for Applied Microbiology and Research, Porton Down, Salisbury Wiltshire SP4 0JG United Kingdom, under No 90020103 on 1st February 1990.

### Example 3

Kendrick test. This was performed according to W.H.O. Requirements for Pertussis Vaccine using outbred NIH-S mice (OLAC, category 3, free of most pathogens including B.bronchiseptica), weighting 14-16g. The antigens, in 0.5ml volumes, were inoculated intraperitoneally as a mixture, and comprised a top dilution and three three-fold serial dilutions. After two weeks the mice were challenged intracerebrally using the recommended challenge strain 18-323 (-400 LD₅₀). The number of survivors in each group was used for calculation of the relative potency in respect to the British Pertussis Reference Vaccine 66/84 using a program of parallel line probit analysis. A comparative test was also preformed using an 69kDa/LPF vaccine. The results are shown in Table 1. In this experiment the 69kDa protein and FHA originated from the strain BP357.

**TABLE 1**

| **69kd (µg)** | **FHA (µg)** | **SURVIVORS** |
|---|---|---|
| 20 | 20 | 13/16 |
| 6.7 | 6.7 | 12/16 |
| 2.2 | 2.2 | 8/16 |
| 0.74 | 0.74 | 8/16 |

| **69kd (µg)** | **LPF (ng)** | |
|---|---|---|
| 20 | 100 | 8/16 |
| 6.7 | 33 | 6/16 |
| 2.2 | 11 | 0/16 |
| 0.74 | 3.7 | 1/16 |

| **66/84** | **I.U.** | |
|---|---|---|
| Whole cell UK | 0.25 | 9/15 |
| reference | 0.08 | 9/16 |
| vaccine | 0.028 | 2/16 |
| | 0.009 | 1/16 |

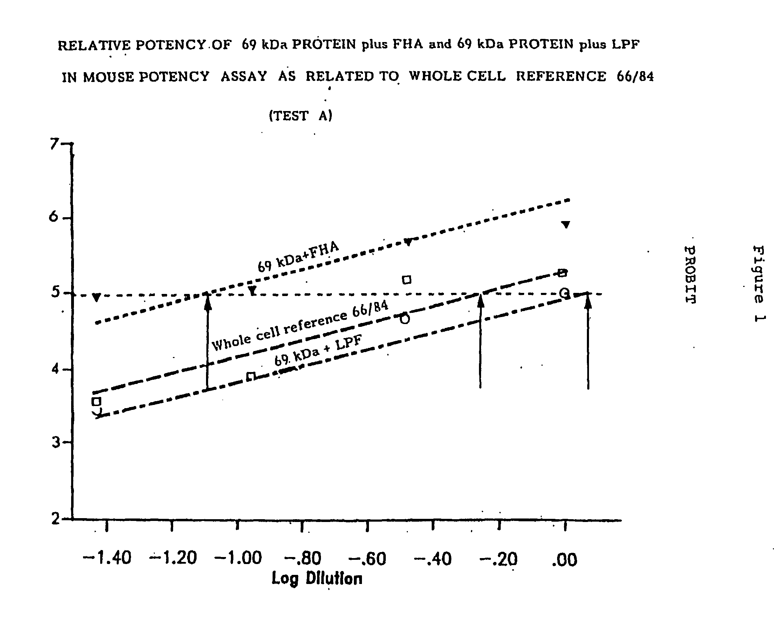

This data was computed according to the methodology of parallel line probits analysis. The results are displayed in Figure 1.

The results clearly show that the 69kDa/FHA vaccine is more potent than 69kDa/LPF and at least as, if not more, potent than the whole cell vaccine.

### Example 4

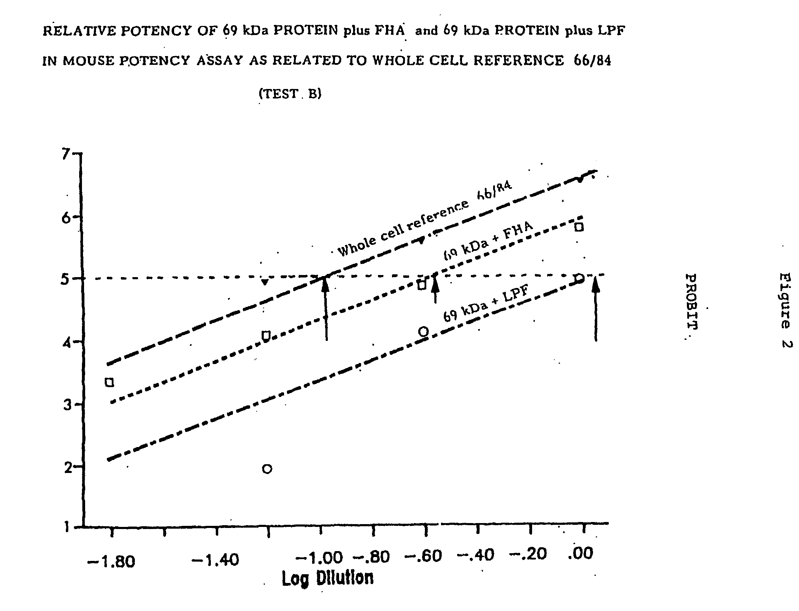

Combinations and/or individual antigens originating from the strain W28ΔLPF of Bordetella pertussis were used in another Kendrick test. Since in this strain the whole toxin gene has been blocked, the possibility that these preparations were contaminated by LPF is nil. The antigen(s) in 0.5ml volumes were inoculated intraperitoneally, individually or as mixtures and comprised a top dose and three four-fold dilutions: After two weeks the mice were challenged intracerebrally using a challenge strain 18-323 (ca 400 LD50). The number of survivors in each group was used for the calculation of the relative potency in respect of the British Pertussis Reference Vaccine 66/84 using a program of parellel line probit analysis. The British Reference 66/84 was used at top dilution containing 0.5 I.U. and three four-fold serial dilutions. The results are shown in table 2 and figure 2.

**TABLE 2**

| **69kDa (ug)** | **FHA (ug)** | **LPF (ng)** | **SURVIVORS/TOTAL** |
|---|---|---|---|
| 20.0 | - | - | 0/18 |
| 5.0 | - | - | 1/18 |
| 1.25 | - | - | 1/18 |
| 0.325 | - | - | 0/18 |
| 20.0 | - | 100.0 | 8/17 |
| 5.0 | - | 20.0 | 3/17 |
| 1.25 | - | 5.0 | 0/20 |
| 0.325 | - | 1.25 | 0/18 |
| 20.0 | 20.0 | - | 14/18 |
| 5.0 | 5.0 | - | 8/18 |
| 1.25 | 1.25 | - | 3/18 |
| 0.325 | 0.325 | - | 1/18 |
| - | 20.0 | - | 0/17 |
| - | 5.0 | - | 6/18 |
| - | 1.25 | - | 4/18 |
| - | 0.325 | - | 1/17 |

| Whole cell reference 66/84 | | | |
|---|---|---|---|
| 0.5 I.U. | | | 16/17 |
| 0.125 | | | 13/18 |
| 0.031 | | | 8/17 |
| 0.008 | | | 0/18 |

| Challenge titration: | | | |
|---|---|---|---|
| Challenge dose | | | 0/11 |
| Challenge dose 1/50 | | | 1/10 |
| Challenge dose 1/250 | | | 2/10 |
| Challenge dose 1/1250 | | | 6/9 |

## Claims (Claims for the following Contracting State(s): AT, BE, CH, DE, DK, FR, GB, IT, LI, LU, NL, SE)

1. An acellular vaccine comprising the 69kDa antigen of Bordetella pertussis, the filamentous haemagglutinin antigen of Bordetella pertussis, an adjuvant and a saline solution, the 69kDa antigen and the filamentous haemagglutinin antigen being present in a weight ratio of between 1:10 and 1:1 and the vaccine being at least as potent as British reference whole cell vaccine 66/84 in the Kendrick test.

2. A vaccine as claimed in claim 1 which is devoid of the B. pertussis toxin.

3. A vaccine as claimed in claim 1 or 2 wherein the ratio is approximately 1:1.

4. A vaccine as claimed in any one of claims-1 to 3 wherein the concentration of antigenic protein is in the range of from 0.01 to 5.0 mg/ml.

5. A vaccine as claimed in any one of claims 1 to 4 for use in a method of treatment of the human or animal body by therapy.

6. A vaccine as claimed in any one of claims 1 to 4 for use in a method of inducing immunity to whooping cough in man.

7. Use of the 69kDa antigen of Bordetella pertussis, the filamentous haemagglutinin antigen of Bordetella pertussis, an adjuvant and a saline solution for the manufacture of an acellular vaccine for the prophylactic treatment of a mammal susceptible to B. pertussis infection, wherein the vaccine contains the 69kDa antigen and the filamentous haemagglutinin antigen in a weight ratio of between 1:10 and 1:1 and is at least as potent as British reference whole cell vaccine 66/84 in the Kendrick test.

8. A use according to claim 7 wherein the vaccine is devoid of the B. pertussis toxin.

9. A use according to claim 7 or 8 wherein the vaccine contains the 69kDa antigen and the filamentous haemagglutinin antigen in a weight ratio of approximately 1:1.

10. A use according to claim 7, 8 or 9 wherein the concentration of antigenic protein in the vaccine is in the range of from 0.01 to 5.0 mg/ml.

11. A method for the preparation on an acellular vaccine, which method comprises mixing the 69kDa antigen of Bordetella pertussis, the filamentous haemagglutinin antigen of Bordetella pertussis, an adjuvant and a saline solution in amounts that provide the 69kDa antigen and the filamentous haemagglutinin antigen in a weight ratio of between 1:10 and 1:1, wherein the vaccine is at least as potent as British reference whole cell vaccine 66/84 in the Kendrick test.

12. A method according to claim 11 wherein the vaccine is devoid of the B. pertussis toxin.

13. A method according to claim 11 or 12 wherein the 69kDa antigen and the filamentous haemagglutinin antigen are provided in a weight ratio of approximately 1:1.

14. A method according to claim 11, 12 or 13 wherein the antigenic protein is provided in a concentration in the range of from 0.01 to 5.0 mg/ml.

## Claims (Claims for the following Contracting State(s): ES)

1. A method for the preparation of an acellular vaccine for the prophylactic treatment of a mammal susceptible to B. pertussis infection, which method comprises mixing the 69kDa antigen of Bordetella pertussis, the filamentous haemagglutinin antigen of Bordetella pertussis, an adjuvant and a saline solution in amounts that provide the 69kDa antigen and the filamentous haemagglutinin antigen in a weight ratio of between 1:10 and 1:1, wherein the vaccine is at least as potent as British reference whole cell vaccine 66/84 in the Kendrich test.

2. A method according to claim 1 wherein the vaccine is devoid of the B. pertussis toxin.

3. A method according to claim 1 or 2 wherein the 69kDa antigen and the filamentous haemagglutinin antigen are provided in a weight ratio of approximately 1:1.

4. A method according to claim 1, 2 or 3 wherein the antigenic protein is provided in a concentration in the range of from 0.01 to 5.0 mg/ml.

5. Use of the 69kDa antigen of Bordetella pertussis, the filamentous haemagglutinin antigen of Bordetella pertussis, an adjuvant and a saline solution for the manufacture of an acellular vaccine for the prophylactic treatment of a mammal susceptible to B. pertussis infection, wherein the vaccine contains the 69kDa antigen and the filamentous haemagglutinin antigen in a weight ratio of between 1:10 and 1:1 and is at least as potent as British reference whole cell vaccine 66/84 in the Kendrick test.

6. A use according to claim 5 wherein the vaccine is devoid of the B. pertussis toxin.

7. A use according to claim 5 or 6 wherein the vaccine contains the 69kDa antigen and the filamentous haemagglutinin antigen in a weight ratio of approximately 1:1.

8. A use according to claim 5, 6 or 7 wherein the concentration of antigenic protein in the vaccine is in the range of from 0.01 to 5.0 mg/ml.

9. An acellular vaccine comprises the 69kDa antigen of Bordetella pertussis, the filamentous haemagglutinin antigen of Bordetella pertussis, an adjuvant and a saline solution, the 69kDa antigen and the filamentous haemagglutinin antigen being present in a weight ratio of between 1:10 and 1:1 and the vaccine being at least as potent as British reference whole cell vaccine 66/84 in the Kendrick test.

10. A vaccine as claimed in claim 9 which is devoid of the B. pertussis toxin.

11. A vaccine as claimed in claim 9 or 10 wherein the ratio is approximately 1:1.

12. A vaccine as claimed in any one of claims 9 to 11 wherein the concentration of antigenic protein is in the range of from 0.01 to 5.0 mg/ml.

13. A vaccine as claimed in any one of claims 9 to 12 for use in a method of treatment of the human or animal body by therapy.

14. A vaccine as claimed in any one of claims 9 to 12 for use in a method of inducing immunity to whooping cough in man.

## Patentansprüche (Patentansprüche für folgende(n) Vertragsstaat(en): AT, BE, CH, DE, DK, FR, GB, IT, LI, LU, NL, SE)

1. Azellulärer Impfstoff, welcher das 69kDa-Antigen von Bordetella pertussis, das filamentöse Hämagglutinin-Antigen von Bordetella pertussis, ein Adjuvans und eine Kochsalzlösung umfasst, wobei das 69kDa-Antigen und das filamentöse Hämagglutinin-Antigen in einem Gewichtsverhältnis von 1:10 bis 1:1 vorliegen und der Impfstoff mindestens so potent ist wie der Britische Referenz-Ganzkeimlmpfstoff 66/84 im Kendrick-Test.

2. Impfstoff nach Anspruch 1, welchem das B. pertussis-Toxin fehlt.

3. impfstoff nach Anspruch 1 oder 2, bei welchem das Verhältnis etwa 1:1 beträgt.

4. Impfstoff nach einem der Ansprüche 1 bis 3, wobei die Konzentration an antigenem Protein im Bereich von 0,01 bis 5,0 mg/ml liegt.

5. Impfstoff nach einem der Ansprüche 1 bis 4 zur Verwendung bei einer Methode zur Behandlung des menschlichen oder tierischen Körpers durch eine Therapie.

6. Impfstoff nach einem der Ansprüche 1 bis 4 zur Verwendung bei einer Methode zur Induzierung einer Immunität gegen Keuchhusten beim Menschen.

7. Verwendung des 69kDa-Antigens von Bordetella pertussis, des filamentösen Hämagglutinin-Antigens von Bordetella pertussis, eines Adjuvans und einer Kochsalzlösung zur Herstellung eines azellulären Impfstoffes für die prophylaktische Behandlung eines für eine B. pertussis-Infektion empfänglichen Säugers, wobei der Impfstoff das 69kDa-Antigen und das filamentöse Hämagglutinin-Antigen in einem Gewichtsverhältnis von 1:10 bis 1:1 enthält und er mindestens so potent ist wie der Britische Referenz-Ganzkeim-Impfstoff 66/84 im Kendrick-Test.

8. Verwendung nach Anspruch 7, wobei dem Impfstoff das B. pertussis-Toxin fehlt.

9. Verwendung nach Anspruch 7 oder 8, wobei der Impfstoff das 69kDa-Antigen und das filamentöse Hämagglutinin-Antigen in einem Gewichtsverhältnis von etwa 1:1 enthält.

10. Verwendung nach Anspruch 7, 8 oder 9, wobei die Konzentration an antigenem Protein im Impfstoff im Bereich von 0,01 bis 5,0 mg/ml liegt.

11. Verfahren zur Herstellung eines azellulären Impfstoffes, welches Verfahren das Mischen des 69kDa-Antigens von Bordetella pertussis, des filamentösen Hämagglutinin-Antigens von Bordetella pertussis, eines Adjuvans und einer Kochsalzlösung in Mengen umfasst, so dass das 69kDa-Antigen und das filamentöse Hämagglutinin-Antigen in einem Gewichtsverhältnis von 1:10 bis 1:1 vorliegen, wobei der Impfstoff mindestens so potent ist wie der Britische Referenz-Ganzkeim-Impfstoff 66/84 im Kendrick-Test.

12. Verfahren nach Anspruch 11, wobei dem Impfstoff das B. pertussis-Toxin fehlt.

13. Verfahren nach Anspruch 11 oder 12, wobei das 69kDa-Antigen und das filamentöse Hämagglutinin-Antigen in einem Gewichtsverhältnis von etwa 1:1 vorgesehen sind.

14. Verfahren nach Anspruch 11, 12 oder 13, wobei das antigene Protein in einer Konzentration im Bereich von 0,01 bis 5,0 mg/ml bereitgestellt wird.

## Patentansprüche (Patentansprüche für folgende(n) Vertragsstaat(en): ES)

1. Verfahren zur Herstellung eines azellulären Impfstoffes für die prophylaktische Behandlung eines für eine B. pertussis-Infektion empfänglichen Säugers, welches Verfahren das Mischen des 69kDa-Antigens von Bordetella pertussis, des filamentösen Hämagglutinin-Antigens von Bordetella pertussis, eines Adjuvans und einer Kochsalzlösung in Mengen umfasst, so dass das 69kDa-Antigen und das filamentöse Hämagglutinin-Antigen in einem Gewichtsverhältnis von 1:10 bis 1:1 vorliegen, wobei der Impfstoff mindestens so potent ist wie der Britische Referenz-Ganzkeim-Impfstoff 66/84 im Kendrick-Test.

2. Verfahren nach Anspruch 1, wobei dem Impfstoff das B. pertussis-Toxin fehlt.

3. Verfahren nach Anspruch 1 oder 2, wobei das 69kDa-Antigen und das filamentöse Hämagglutinin-Antigen in einem Gewichtsverhältnis von etwa 1:1 bereitgestellt werden.

4. Verfahren nach Anspruch 1, 2 oder 3, wobei das antigene Protein in einer Konzentration im Bereich von 0,01 bis 5,0 mg/ml vorliegt.

5. Verwendung des 69kDa-Antigens von Bordetella pertussis, des filamentösen Hämagglutinin-Antigens von Bordetella pertussis, eines Adjuvans und einer Kochsalzlösung zur Herstellung eines azellulären lmpfstoffes zur prophylaktischen Behandlung eines für eine B. pertussis-Infektion empfänglichen Säugers, wobei der Impfstoff das 69kDa-Antigen und das filamentöse Hämagglutinin-Antigen in einem Gewichtsverhältnis von 1:10 bis 1:1 enthält und mindestens so potent ist wie der Britische Referenz-Ganzkeim-Impfstoff 66/84 im Kendrick-Test.

6. Verwendung nach Anspruch 5, wobei dem Impfstoff das B.pertussis-Toxin fehlt.

7. Verwendung nach Anspruch 5 oder 6, wobei der Impfstoff das 69kDa-Antigen und das filamentöse Hämagglutinin-Antigen in einem Gewichtsverhältnis von etwa 1:1 enthält.

8. Verwendung nach Anspruch 5, 6 oder 7, wobei die Konzentration an antigenem Protein im Impfstoff im Bereich von 0,01 bis 5,0 mg/ml liegt.

9. Azellulärer Impfstoff, welcher das 69kDA-Antigen von Bordetella pertussis, das filamentöse Hämagglutinin-Antigen von Bordetella pertussis, ein Adjuvans und eine Kochsalzlösung umfasst, wobei das 69kDa-Antigen und das filamentöse Hämagglutinin-Antigen in einem Gewichtsverhältnis von 1:10 bis 1:1 vorliegen und der Impfstoff mindestens so potent ist wie der Britische Referenz-Ganzkeim-Impfstoff 66/84 im Kendrick-Test.

10. impfstoff nach Anspruch 9, welchem das B. pertussis-Toxin fehlt.

11. Impfstoff nach Anspruch 9 oder 10, bei welchem das Verhältnis etwa 1:1 beträgt.

12. Impfstoff nach einem der Ansprüche 9 bis 11, wobei die Konzentration an antigenem Protein im Bereich von 0,01 bis 5,0 mg/ml liegt.

13. Impfstoff nach einem der Ansprüche 9 bis 12 zur Verwendung bei einer Methode zur Behandlung des menschlichen oder tierischen Körpers durch eine Therapie.

14. Impfstoff nach einem der Ansprüche 9 bis 12 zur Verwendung bei einer Methode zur Induzierung einer Immunität gegen Keuchhusten beim Menschen.

## Revendications (Revendications pour l'(les) Etat(s) contractant(s) suivant(s): AT, BE, CH, DE, DK, FR, GB, IT, LI, LU, NL, SE)

1. Vaccin acellulaire comprenant l'antigène de 69 kDa de Bordetella pertussis, l'antigène de l'hémagglutinine filamenteuse de Bordetella pertussis, un adjuvant et une solution saline, l'antigène de 69 kDa et l'antigène de l'hémagglutinine filamenteuse étant présents dans un rapport pondéral compris entre 1 : 10 et 1 :1 et le vaccin étant au moins aussi puissant que le vaccin britannique de référence avec cellules entières 66/84 dans le test de Kendrick.

2. Vaccin tel que revendiqué dans la revendication 1 qui est exempt de la toxine de B. pertussis.

3. Vaccin tel que revendiqué dans la revendication 1 ou 2 dans lequel le rapport est approximativement de 1 : 1.

4. Vaccin tel que revendiqué dans l'une quelconque des revendications 1 à 3 dans lequel la concentration de protéine antigénique est dans la gamme de 0,01 à 5,0 mg/ml.

5. Vaccin tel que revendiqué dans l'une quelconque des revendications 1 à 4 utilisable dans une méthode de traitement du corps humain ou animal par thérapie.

6. Vaccin tel que revendiqué dans l'une quelconque des revendications 1 à 4 utilisable dans une méthode d'induction de l'immunité contre la coqueluche chez l'homme.

7. Utilisation de l'antigène de 69 kDa de Bordetella pertussis, de l'antigène de l'hémagglutinine filamenteuse de Bordetella pertussis, d'un adjuvant et d'une solution saline pour la préparation d'un vaccin acellulaire pour le traitement prophylactique d'un mammifère sensible à une infection par B. pertussis, dans laquelle le vaccin contient l'antigène de 69 kDa et l'antigène de l'hémagglutinine filamenteuse dans un rapport pondéral compris entre 1 : 10 et 1 : 1 et est au moins aussi puissant que le vaccin britannique de référence avec cellules entières 66/84 dans le test de Kendrick.

8. Utilisation selon la revendication 7 dans laquelle le vaccin est exempt de la toxine de B. pertussis.

9. Utilisation selon la revendication 7 ou 8 dans laquelle le vaccin contient l'antigène de 69 kDa et l'antigène de l'hémagglutinine filamenteuse dans un rapport pondéral d'approximativement 1 : 1.

10. Utilisation selon la revendication 7, 8 ou 9 dans laquelle la concentration de protéine antigénique dans le vaccin est dans la gamme de 0,01 à 5,0 mg/ml.

11. Méthode de préparation d'un vaccin acellulaire, méthode qui comprend le fait de mélanger l'antigène de 69 kDa de Bordetella pertussis, l'antigène de l'hémagglutinine filamenteuse de Bordetella pertussis, un adjuvant et une solution saline dans des quantités qui fournissent l'antigène de 69 kDa et l'antigène de l'hémagglutinine filamenteuse dans un rapport pondéral compris entre 1 : 10 et 1 : 1, dans laquelle le vaccin est au moins aussi puissant que le vaccin britannique de référence avec cellules entières 66/84 dans le test de Kendrick.

12. Méthode selon la revendication 11 dans laquelle le vaccin est exempt de la toxine de B. pertussis.

13. Méthode selon la revendication 11 ou 12 dans laquelle le vaccin de 69 kDa et l'antigène d'hémagglutinine filamenteuse sont fournis dans un rapport pondéral d'approximativement 1 : 1.

14. Méthode selon la revendication 11, 12 ou 13 dans laquelle la protéine antigénique est fournie dans une concentration dans la gamme de 0,01 à 5,0 mg/ml.

## Revendications (Revendications pour l'(les) Etat(s) contractant(s) suivant(s): ES)

1. Méthode de préparation d'un vaccin acellulaire pour le traitement prophylactique d'un mammifère sensible à une infection par B. pertussis, méthode qui comprend le fait de mélanger l'antigène de 69 kDa de Bordetella pertussis, l'antigène de l'hémagglutinine filamenteuse de Bordetella pertussis, un adjuvant et une solution saline en des quantités qui fournissent l'antigène de 69 kDa et l'antigène de l'hémagglutinine filamenteuse dans un rapport pondéral compris entre 1 : 10 et 1 :1, dans laquelle le vaccin est au moins aussi puissant que le vaccin britannique de référence avec cellules entières 66/84 dans le test de Kendrick.

2. Méthode selon la revendication 1 dans laquelle le vaccin est exempt de la toxine de B. pertussis.

3. Méthode selon la revendication 1 ou 2 dans laquelle l'antigène de 69 kDa et l'antigène de l'hémagglutinine filamenteuse sont fournis dans un rapport pondéral d'approximativement 1 : 1.

4. Méthode selon la revendication 1, 2 ou 3 dans laquelle la protéine antigénique est fournie dans une concentration dans la gamme de 0,01 à 5,0 mg/ml.

5. Utilisation de l'antigène de 69 kDa de Bordetella pertussis, de l'antigène de l'hémagglutinine filamenteuse de Bordetella pertussis, d'un adjuvant et d'une solution saline pour la préparation d'un vaccin acellulaire pour le traitement prophylactique d'un mammifère sensible à une infection par B. pertussis, dans laquelle le vaccin contient l'antigène de 69 kDa et l'antigène de l'hémagglutinine filamenteuse dans un rapport pondéral compris entre 1 : 10 et 1 : 1 et est au moins aussi puissant que le vaccin britannique de référence avec cellules entières 66/84 dans le test de Kendrick.

6. Utilisation selon la revendication 5 dans laquelle le vaccin est exempt de la toxine de B. pertussis.

7. Utilisation selon la revendication 5 ou 6 dans laquelle le vaccin contient l'antigène de 69 kDa et l'antigène de l'hémagglutinine filamenteuse dans un rapport pondéral d'approximativement 1 : 1.

8. Utilisation selon la revendication 5, 6 ou 7 dans laquelle la concentration de la protéine antigénique dans le vaccin est dans la gamme de 0,01 à 5,0 mg/ml.

9. Vaccin acellulaire comprenant l'antigène de 69 kDa de Bordetella pertussis, l'antigène de l'hémagglutinine filamenteuse de Bordetella pertussis, un adjuvant et une solution saline, l'antigène de 69 kDa et l'antigène de l'hémagglutinine filamenteuse étant présents dans un rapport pondéral compris entre 1 : 10 et 1 : 1 et le vaccin étant au moins aussi puissant que le vaccin britannique de référence avec cellules entières 66/84 dans le test de Kendrick.

10. Vaccin tel que revendiqué dans la revendication 9 qui est exempt de la toxine de B. pertussis.

11. Vaccin tel que revendiqué dans la revendication 9 ou 10 dans lequel le rapport est approximativement de 1 : 1.

12. Vaccin tel que revendiqué dans l'une quelconque des revendications 9 à 11 dans lequel la concentration de protéine antigénique est dans la gamme de 0,01 à 5,0 mg/ml.

13. Vaccin tel que revendiqué dans l'une quelconque des revendications 9 à 12 utilisable dans une méthode de traitement du corps humain ou animal par thérapie.

14. Vaccin tel que revendiqué dans l'une quelconque des revendications 9 à 12 utilisable dans une méthode d'induction de l'immunité contre la coqueluche chez l'homme.
